# EUROPEAN PATENT APPLICATION

(11) **EP 4 365 280 A1**
(43) Date of publication of application: **08.05.2024**
(21) Application number: 22383051.4
(22) Date of filing: 02.11.2022
(51) Int. Cl.: C12N 1/16, C12P 7/6436, C12P 7/6463

(54) **METHOD FOR CULTURING OLEAGINOUS YEASTS**

(71) Applicant: Fundación IMDEA Energía, 28935 Móstoles (Madrid) (ES)
(72) Inventor: MORALES PALOMO, Sergio, E-28935 Móstoles, Madrid (ES); TOMÁS PEJÓ, Elia, E-28935 Móstoles, Madrid (ES); GONZÁLEZ FERNÁNDEZ, Cristina, E-28935 Móstoles, Madrid (ES)
(74) Representative: ABG Intellectual Property Law, S.L.

(57) **Abstract**

The invention relates to a process for culturing oleaginous yeasts, more particularly of the genus *Yarrowia,* on a culture medium comprising short chain fatty acids (SCFAs) as carbon source and calcium ion (Ca²⁺).

## Description

### FIELD OF THE INVENTION

The present invention relates to a process for culturing oleaginous yeasts, more particularly of the genus *Yarrowia*, on a culture medium comprising short chain fatty acids (SCFAs) as carbon source and calcium ion (Ca²⁺).

### BACKGROUND OF INVENTION

Significant global climate changes and environmental problems have encouraged the replacement of petroleum-based compounds with green and renewable bioproducts. In this sense, animal fats and vegetable oils are regarded as an important green alternative to replace petroderivatives in the chemical industry. Nevertheless, their competition with food necessities, limited supply and the need of using large cultivation areas hampers the oleochemical industry development from these materials. Within this scenario, microbial lipids have been set as a promising source for sustainable production of chemicals and biofuels, due to their fatty acids' composition similarities with vegetable oils.

Several studies have used oleaginous yeasts to obtain lipids due to their ability to accumulate over 60% of lipids w/w per dry weight (DW) using carbon sources derived from organic wastes, such as glycerol, agricultural residues and single chain fatty acids (SCFAs) (Di Fidio et al., 2019, Journal of Microbiology and Biotechnology 29, 256-267). Among all the oleaginous yeasts, *Yarrowia lipolytica* is the most widely studied due to the available knowledge about its genome and numerous alternatives for its genetic modification.

Oleaginous yeasts, more particularly of the species *Yarrowia lipolytica,* are known to be capable, under conditions of nutritional limitation, of consuming carbonaceous sources, such as glucose and glycerol, to produce biomass, to accumulate carbonaceous reserve substances of the type polysaccharides and lipids, and produce exocellular co-products, mainly citric acid.

Sugar-based feedstock, such as glucose and xylose, have been traditionally used as substrates for microbial oil production because they are easily assimilated by most oleaginous yeasts. However, the use of glucose can account for up to 80% of the entire production process (Patel et al, 2018, Molecules, 23(7):1562). In this sense, the utilization of organic wastes as carbon sources will boost the economic viability of the microbial lipids production process. SCFAs are organic acids that can be sustainably produced during the anaerobic fermentation (AF) of wastes after the hydrolytic and acidogenic stages. Although it has been proven that some yeasts can use these SCFAs as a carbon source, the available information about the oleaginous yeasts' metabolism when using SCFAs is still scarce.

The media composition is known to play a major role in yeast growth. Specifically, phosphate (PO₄³⁻) contributes to its growth as is a crucial element for RNA, DNA, phosphorylated proteins and several ubiquitous cofactors. Nevertheless, it has been observed that high concentrations of this anion could inhibit yeast growth when SCFAs were used as carbon source. There are several studies that point to PO₄³⁻ as a calcium (Ca) binder, leaving little availability of this cation in the medium, which could explain the growth inhibition (Chan et al., 2017, Australian Prescriber 40, 10). It is known that certain concentrations of Ca in the medium can help the yeast overcome certain inhibitory barriers such as ethanol and even promote its growth. In this sense, the use of high concentrations of PO₄³⁻ would be detrimental in terms of improving yeast lipid content (% w/w). Moreover, it has been observed that PO4³⁻limitation can affect yeast metabolism to redirect carbon flux towards increased lipid content (% w/w).

Concerns regarding the depletion of natural PO₄³⁻, which is required for fertilizers and appropriate food production, have prompted initiatives to address the high concentrations of PO₄³⁻ present in various wastes. In these cases, the PO₄³⁻ is recovered so that it can be used in other sources (Campos et al., 2019, Frontiers in Sustainable Food Systems 2, 91). Taking this into account, the use of effluents obtained via AF of low-cost residues with low concentrations of PO₄³⁻ could improve both the yeast growth, since the Ca in the medium would be available, as well as the production of lipids. Nevertheless, the knowledge about the effect of Ca on yeast growth and lipid content (% w/w) is still scarce.

### SUMMARY OF THE INVENTION

The authors of the present invention have developed a culture medium that is capable to promote the growth of oleaginous yeasts and their lipid production, in particular yeast of the species *Yarrowia lipolytica.* Said culture medium is characterized in that it comprises short-chain fatty acids (SCFAs) as the main carbon source and calcium. The culture medium also comprises calcium ion (Ca²⁺) and can or cannot comprise phosphate (PO₄³⁻).

Thus, in a first aspect, the invention relates to a method for producing a biomass of an oleaginous yeast comprising culturing the oleaginous yeast with a culture medium comprising SCFAs as carbon source and Ca²⁺, wherein
- the culture medium does not comprise PO₄³⁻ or
- the culture medium comprises PO₄³⁻ and the ratio phosphate/calcium ion (PO₄³⁻: Ca²⁺) is lower than 675
and wherein the culture medium does not comprise more than 2 g/L of glucose.

In a second aspect, the invention relates to a biomass of an oleaginous yeast obtained by the method previously described.

In a third aspect, the invention relates to a method for enriching the lipid content of a biomass of an oleaginous yeast, the method comprising culturing the oleaginous yeast with a culture medium comprising SCFAs as carbon source and Ca²⁺, wherein
- the culture medium not comprise PO₄³⁻ or
- the culture medium comprises PO₄³⁻ and the ratio PO₄³⁻: Ca²⁺is lower than 50 and wherein the culture medium does not comprise more than 2 g/L of glucose.

### DESCRIPTION OF THE FIGURES

**Figure 1****.** *Y. lipolytica* growth and substrate consumption under PO₄³⁻:Ca²⁺ ratios of 336.7 (A), 253.3 (B), 170 (C) and 0 (D) in synthetic media.
**Figure 2****.** *Y. lipolytica* lipid content (% w/w) under different PO₄³⁻:Ca²⁺ ratios in synthetic media.
**Figure 3****.** *Y. lipolytica* growth and substrate consumption under PO₄³⁻:Ca²⁺ ratios of 33.3 (A), 170 (B), 253.3 (C) and 336.7 (D) in RD.
**Figure 4****.** *Y. lipolytica* lipid content (% w/w) under different PO₄³⁻:Ca²⁺ ratios in RD.
**Figure 5****.** *Y. lipolytica* growth and substrate consumption under PO₄³⁻:Ca²⁺ ratios of 336.7 (A), 253.3 (B), 170 (C) and 0 (D) in SM with 20 g/L of SCFAs.
**Figure 6****.** *Y. lipolytica* lipid content (% w/w) under different PO₄³⁻:Ca²⁺ ratios in SM with 20 g/L of SCFAs.
**Figure 7****.** *Y. lipolytica* growth and substrate consumption under PO₄³⁻:Ca²⁺ ratios of 33.3 (A), 170 (B), 253.3 (C) and 336.7 (D) in real media with 20 g/L of SCFAs.
**Figure 8****.** *Y. lipolytica* lipid content (% w/w) under different PO₄³⁻:Ca²⁺ ratios in real media with 20 g/L of SCFAs.
**Figure 9****.** Time course of *Y. lipolytica* growth and SCFAs consumption in RD (A and B) with different total PO₄³⁻ concentrations.

### DESCRIPTION OF THE INVENTION

### Culture medium of the invention

The authors of the invention have discovered that the PO₄³⁻:Ca²⁺ ratio in the culture medium influences the growth of oleaginous yeasts and their lipid production when using SCFAs as the main carbon source, so they have developed a method for producing a biomass of an oleaginous yeast, in particular of the species *Yarrowia lipolytica,* which comprises culturing the oleaginous yeast with a culture medium. Said culture medium comprises at least a SCFAs as the main carbon source and Ca²⁺.

Thus, in a first aspect, the invention relates to a method for producing a biomass of an oleaginous yeast (hereinafter "the first method of the invention") comprising culturing the oleaginous yeast with a culture medium comprising SCFAs as carbon source and Ca²⁺, wherein
- the culture medium does not comprise PO₄³⁻ or
- the culture medium comprises PO₄³⁻ and the ratio PO₄³⁻: Ca²⁺ is lower than 675 and wherein the culture medium does not comprise more than 2 g/L of glucose.

The term, "culture" or "cultivation" refers to the maintenance or proliferation of cells in an artificial *in vitro* environment. The culture media can be used for the culture of any oleaginous yeast, especially oleaginous yeast from the species *Yarrowia lipolytica.*

The term "oleaginous yeast" as used herein, refers to oleaginous yeast species with oil contents exceeding of 20% w/w biomass dry weight. Oleaginous yeasts are able to produce and accumulate more than 20% of lipids w/w per dry weight (DW). Examples of oleaginous yeasts include, but are not limited to: *Y. lipolytica, Lipomyces starkey, Rhodotorula toruloides, Rhodotorula glutinis, Trichosporon fermentans,* and *Trichosporon oleaginosus.* There are other oleaginous microorganisms, as filamentous fungi or microalgae, but yeasts exhibit advantages for lipid production over other oleaginous microorganisms, due to their unicellular form, short duplication times, capability to grow on a large variety of raw materials, and their easy cultivation in large fermenters.

In a particular embodiment, the oleaginous yeast is selected from the group consisting of: *Y. lipolytica, Cutaneotrichosporon oleaginosus* (previously known as *T. oleaginosus, Cryptococcus curvatus, Apiotrichum curvatum* or *Candida curvata*) *R. toruloides, Rhodotorula mucilaginosa, Rhodotorula mucilaginosa var. mucilaginosa, Rhodotorula terpenoidalis, Sporobolomyces alborubescens, Starmerella bombicola, Torulaspora delbruekii, Torulaspora pretoriensis, Lipomyces lipofer, L. starkey, Debaryomyces hansenii, Candida lipolytica, Candida glabrata, Candida tropicalis, Hansenula polymorpha, Hansenula californica, Hansenula beijerinckii, Geotrichum silvicola, Geotrichum vulgare* and *Williopsis saturnus.*

In a more particular embodiment, the oleaginous yeast is from the species *Y. lipolytica.* The term *"Yarrowia"* as used herein, refers to a genus of *Ascomycetous* yeast in the family *Dipodascaceae.* The term "*Y. lipolytica"* refers to a species of the genus *Yarrowia*, which is widespread in nature and has considerable industrial utility as well as importance to the food and medical fields. Due to *Y. lipolytica's* unique physiological characteristics (i.e., its ability to metabolize hydrophobic substrates such as alkanes, fatty acids, and lipids), its ability to accumulate high levels of lipids, and its suite of efficient genetic tools, this yeast is a model organism for lipid production and it is thought to have great applied potential, both in the production of typical biofuel lipids and oils with unusual fatty acid profiles or polyunsaturated fatty acids. *Y. lipolytica* is able to accumulate more than 20% w/w lipids per DW.

The first method of the invention does not only concern the cultivation of wild-type strains of oleaginous yeasts (natural yeasts), it also contemplates the cases of culturing mutant oleaginous yeasts obtained from a mutagenic treatment. Note that, the term "wild-type strain" refers to a yeast which has been originally present in nature, in other words, a yeast to the gene(s) of which has(have) not been applied any artificial mutagenic treatment. On the other hand, the term "mutant strain" means a yeast obtained by applying an artificial mutagenic treatment to the gene(s).

The mutagenic treatment in the present invention is not specifically limited as long as it is a treatment that can cause a mutation in a part of gene(s) of a yeast. Any usual technique for use in the production of mutant strains of a yeast may be employed. For example, a mutagenic treatment onto a yeast can be carried out by treating the yeast with, as a mutagen, ultraviolet radiation, ionizing radiation, a nitrite, nitrosoguanidine or ethyl methane sulfonate (hereunder, abbreviated as EMS).

The term "biomass" as used herein, refers to biological material comprising, living or recently living organisms. By extension, the term includes not only the biological material or organic matter which constitutes an organism, but also the biological material or organic matter generated in a biological process, spontaneous or not spontaneous (i.e., provoked). Biomass can be determined as the total quantity or weight of oleaginous yeasts in a given area or volume. Biomass is often reported as a mass per volume of medium, typically g DW/L and usually as dry weight (water removed by drying).

The term "carbon source" as used herein, refers to the molecules used by the oleaginous yeast as the source of carbon for building its biomass. A carbon source can be an organic compound or an inorganic compound.

The method of the invention comprises using SCFAs as the main carbon source.

However, the culture medium may comprise other carbon sources. The additional carbon source of the culture medium may be exemplified by one or more materials selected from the group consisting of glucose, sucrose, acetic acid, ethanol, molasses, spent sulphite liquor and the like, which, as a person skilled in the art may know, are used for routine cultivation of a microorganism. However, in particular embodiment, the culture medium does not comprise other carbon source.

In a particular embodiment, if the culture medium comprises glucose as an additional carbon source, it does not comprise more than 2 g/L of glucose. In a more particular embodiment the culture medium does not comprise more than 1 g/L, more than 0,75 g/L, more than 0,50 g/L, more than 0,25 g/L, more than 0,01 g/L of glucose.

Additionally, the culture medium may comprise other components like a nitrogen source and an inorganic salt.

The nitrogen source may be either a nitrogen-containing inorganic salt or a nitrogen-containing organic material. For example, one or more materials selected from the group consisting of urea, ammonia, ammonium sulfate, ammonium chloride, ammonium phosphate, corn steep liquor (CSL), casein, yeast extract, peptone, and the like, can be used. Moreover, the inorganic salt can be exemplified by a phosphate component such as calcium superphosphate or ammonium phosphate, a potassium component such as potassium chloride or potassium hydroxide, a magnesium component such as magnesium sulfate or magnesium chloride. In addition, an inorganic salt of zinc, copper, manganese, ferrous ion, or the like, may also be used. Furthermore, the culture medium can comprise vitamins.

The term "vitamin" or "essential nutrient" as used herein refers to an organic molecule (or related set of molecules) that is an essential micronutrient that an organism needs in small quantities for the proper functioning of its metabolism. Essential nutrients cannot be synthesized in the organism, either at all or not in sufficient quantities, and therefore must be obtained through the diet. The term vitamin does not include the three other groups of essential nutrients: minerals, essential fatty acids, and essential amino acids. As mentioned above, the first method of the invention comprises culturing the oleaginous yeast with a culture medium comprising at least SCFAs as carbon source and Ca. Before inoculating the culture medium with oleaginous yeast, the first method of the invention can comprise a previous step which consist in the culture of the oleaginous yeast in a nutritive medium with the objective of generate an initial quantity of cells that will be used to inoculate the culture medium. Thus, in a particular embodiment, the first method of the invention comprises a previous step of culturing the yeasts until they reached the late exponential growth phase.

The term "exponential growth phase" or "logarithmic growth phase" as used herein, refers to a phase in which a logarithmic increase of the amount of a yeast in a culture vessel is observed if measured in a time course manner with reference to the absorbance as an index.

In this previous step, the oleaginous yeasts are cultivated in a nutritive medium. The term "nutritive medium" as used herein, refers to a liquid or solid formulation containing nutrients and other conditions necessary for the growth of the oleaginous yeasts. As an expert in the field may know, the culture of the oleaginous yeast until it reached the exponential growth phase can be done in different nutritive mediums. For example, it can be used PDA, YEPD or YPD mediums. In a particular embodiment of the present invention, the nutritive medium is YPD (yeast extract-peptone-dextrose). The YPD nutritive medium can be liquid or solid. The YPD solid medium contains yeast extract, peptone, dextrose and agar. The YPD liquid medium contains yeast extract, peptone and dextrose.

In a particular embodiment, the culture of the oleaginous yeast with the culture medium is made at a temperature of between 20°C and 30°C (both ends included) in stirred conditions, and at an initial pH of between 5.0 - 8.0 (both ends included). In a more particular embodiment, the culture is done at 27°C and at initial pH of 6. The culture of the oleaginous yeast with the culture medium can be done in pH-uncontrolled culture conditions or in pH-controlled culture conditions. The pH-controlled culture conditions refers to the use of a pH indicator and comprises the use a chemical buffering system or a buffering agent. The terms "buffer," "buffering system," and/or "buffer solution" refer to a solution, which reduces the change of pH upon addition of small amounts of acid or base, or upon dilution. In a particular embodiment of the present invention, the culture of the oleaginous yeast with the culture medium is done in pH-uncontrolled culture conditions.

The term "stirred conditions" as used herein, refers to culture conditions in which the culture medium, and the oleaginous yeast contained therein as well, are in motion, preferably in motion with a stable frequency.

The stirred conditions can be appropriately determined with consideration of the volume, the duration of the culture, and the initial concentration of the oleaginous yeast. In a particular embodiment, stirred conditions are realised at approximately 10, 20, 30, 40, 50, 55, 60, 65, 70, 75, 80, 85, 90, 96, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 260, 270, 280, 290, 300, 310, 320, 350, 370, 400, 450, 500, 550, 600, 650, 700, 750, 750, 800, 850, 900, 1000 rpm, preferably the stirred conditions are realised at 170 rpm.

The stirred conditions can be realised by methods well known to a person skilled in the art, such as placing the culture in a shake flask, a rotary shaker, a stirred bioreactor, a flask placed on a shaker platform or a rotator. In a particular embodiment of the present invention, stirred culture conditions are carried out with a rotary shaker.

In another particular embodiment, the oleaginous yeast is cultivated in the culture medium in stirred conditions until more than the 75-80% of the SCFAs were consumed, preferably until more than 85-90% of the SCFAs were consumed, more preferably until 95-100% of the SCFAs were consumed.

As an expert in the field may know, many different instrumental techniques can be used to determine the SCFAs concentration in the culture medium; these techniques include gas chromatography (GC), gas chromatography-mass spectrometer (GC-MS), liquid chromatograph (LC), capillary electrophoresis (CE), ion exchange high-performance liquid chromatography (IC) and nuclear magnetic resonance (NMR). In a particular embodiment of the present invention, the SCFAs concentration in the culture medium is measured using the liquid chromatography (LC).

In another particular embodiment, upon inoculation of the culture medium with the oleaginous yeast, an initial optical density (OD) at a wavelength of 600 nm is set of about 0.5, 0.6, 0.7, 0.8, 0.9, 1, 2, 3, 4, 5, 6, 7, preferably about 1 (equivalent to 0.45 g DW cells/L).

The carbon source in the culture medium according to the first method of the invention is one or more SCFAs. SCFAs are fatty acids with fewer than six carbon atoms. SCFAs all possess varying degrees of water solubility, which distinguishes them from longer chain fatty acids that are immiscible. SCFAs are organic acids that can be sustainably produced during the anaerobic fermentation (AF) of wastes after the hydrolytic and acidogenic stages. Some examples of SCFAs are formic acid acetic acid, propionic acid, butyric acid, isobutyric acid, valeric acid, isovaleric acid, 2-methylbutyric acid and hexanoic acid.

In a particular embodiment of the present invention, the SCFAs of the culture medium are selected from the group consisting of acetic acid, propionic acid, butyric acid, valeric acid, hexanoic acid and combination thereof.

Acetic acid, systematically named ethanoic acid, is an acidic, colourless liquid and organic compound with the chemical formula CH₃COOH. Acetic acid is the second simplest carboxylic acid (after formic acid) and its functional group is methyl.

Propionic acid, also known as propionic acid, is a naturally occurring carboxylic acid with chemical formula CH₃CH₂CO₂H.

Butyric acid, also known under the systematic name butanoic acid, is a straight-chain alkyl carboxylic acid with the chemical formula CH₃₍CH₂₎₂CO₂H.

Valeric acid or pentanoic acid is a straight-chain alkyl carboxylic acid with the chemical formula CH₃(CH₂)₃COOH.

Hexanoic acid or caproic acid, is the carboxylic acid derived from hexane with the chemical formula CH₃(CH₂)₄COOH.

In a particular embodiment of the present invention, the carbon source of the culture medium is a combination of SCFAs, preferably a combination of acetic acid, propionic acid, butyric acid, valeric acid and hexanoic acid. In a particular embodiment of the present invention, the culture medium comprises at least 10 g/L of total SCFAs. In a more particular embodiment, the culture medium comprises at least 20 g/L of total SCFAs.

In a particular embodiment, the concentration of the acetic acid is between 2 and 20 g/L, preferably between 2.5 and 10 g/L (all ends included).

In another particular embodiment, the concentration of the propionic acid is between 0.5 and 10 g/L, preferably between 1 and 4 g/L (all ends included).

In another particular embodiment, the concentration of the butyric acid is between 5 and 15 g/L, preferably between 5.5 and 9 g/L (all ends included).

In another particular embodiment, the concentration of the valeric acid is between 1.5 and 10 g/L, preferably between 2 and 5 g/L (all ends included).

In another particular embodiment, the concentration of the hexanoic acid is between 2 and 10 g/L, preferably between 2 and 5 g/L (all ends included).

The culture medium can or cannot comprise phosphate (PO₄³⁻). When PO₄³⁻ is present in the culture medium, the ratio PO₄³⁻: Ca²⁺ is lower than 675. In a particular embodiment the ratio PO₄³⁻: Ca²⁺ is lower than 350, lower than 300, lower than 250, lower than 200, lower than 150, lower than 100, lower than 50, or lower than 25, preferably the ratio PO₄³⁻: Ca²⁺is comprised between 30 and 350. In a particular embodiment, the ratio PO₄³⁻: Ca²⁺is about 336.7, about 253.3, about 170 or about 33.33.

The term "the ratio PO₄³⁻: Ca²⁺" as used herein, refers to the values obtained from the division of the PO₄³⁻ concentration between the Ca²⁺ concentration. In the context of the present invention, the term "calcium" or "Ca" is used as a synonym of "calcium ion" or "Ca²⁺". The Ca²⁺ and the PO₄³⁻ can be added in the form of a salt, for example, calcium chloride, calcium acetate, calcium citrate, calcium gluconate or others for calcium or. NaH₂PO₄, Na₂HPO₄, KH₂PO₄ and K₂HPO₄ for phosphate. In a particular embodiment, the ratios PO₄³⁻: Ca²⁺ are obtained by adding Ca, preferably in the form of CaCl₂, and PO₄³⁻, preferably in the form of KH₂PO₄, to the culture medium. In a more particular embodiment, the culture medium is supplemented with a calcium concentration varying between 0.01 and 0.05 g/L, preferably 0.03 g/L (corresponding to 0.09 g/L of CaCl₂). It will be understood that the concentrations of these components, while being present at concentration ratios as defined above, should also be present at concentrations that do not result in a substantially toxicity to the cells.

The expression "concentration that does not result in a substantial toxicity to the cells" as used herein, refers to the concentration of a component in a culture medium that does not substantially delay the growth of the cells in comparison with the growth in the same medium and under the same condition in the absence of the component. The concentration of a given component that does not result in a substantial toxicity to the cells prevent the cells to grow when present in a culture medium can determined by calculating the IC50 for said component, said IC50 being the concentration that causes a 50% growth inhibition.

As a person skilled in the field understands, after the culture of the oleaginous yeast with the culture medium, or after the fermentation process, the oleaginous yeast or the biomass of the oleaginous yeast can be recovered from the culture product by a known method. The method of recovering the yeast or the biomass of the yeast from the culture product is not specifically limited, and any recovery method which is usually conducted for recovering a yeast from a culture product of the yeast can be adopted. The method of recovering the yeast or the biomass of the yeast from the culture product can be exemplified by a method of centrifuging and filtrating the culture product.

The term "fermentation" refers to a metabolic process that produces chemical changes in organic substrates through the action of enzymes. The fermentation process can have various purposes, i.e. to increase cell biomass, to produce enzymes or to produce metabolites.

The term "culture product of the yeast" as used herein, refers to a composition obtained from the fermentation of the culture medium by the oleaginous yeasts, and comprising active compounds as a result of the enzymatic activity of the yeasts on the culture medium.

As a person skilled in the field understands, the biomass of the yeast recovered from the culture product can be further processed. Thus, the obtained oleaginous yeast biomass can be washed with distilled water or NaCl at 0.88% one or more times, preferably more than 2 times, and can be dried. The method of drying is not specifically limited, and any method which is usually conducted for preparing dry yeast cells or dry yeast biomass can be adopted. The method of drying can be exemplified by the freeze dry method, the lyophilization method, heat dry method, or the like.

In addition to obtaining the oleaginous yeast biomass, a yeast extract can also be obtained from the culture product of the yeast. The term "yeast extract" as used herein refers to the extract obtained after extraction, filtration and/or concentration of the product obtained after the fermentation process. The term "extract" or "extraction" as used herein, refers to the process by which the active compounds retained inside the cell, in particular the oleaginous yeasts, are released into the medium.

The preparation of a yeast extract from the oleaginous yeast cultured by the first method of the invention is not specifically limited, and any preparation method which is usually conducted for preparing a yeast extract can be used. The preparation method can be exemplified by the autolysis method of lysing cell bodies with the aid of proteases that are naturally present in the yeast cells, the enzymatic degradation method of lysing cell bodies by adding an enzyme preparation derived from a microorganism or a plant, the hot water extraction method of lysing cell bodies by soaking cells in hot water for a fixed period of time, the acid/alkali decomposition method of lysing cell bodies by adding various types of acids or alkalis, the freezing and thawing method of disrupting cell bodies by conducting one or more times of freezing and thawing, the physical disruption method of disrupting cell bodies by a physical stimulation, or the like. The physical stimulation to be used in the physical disruption method can be exemplified by ultrasonic treatment, homogenization under high pressure, grinding by mixing with a solid material such as glass beads, or the like.

After the extraction process, the resulting product is filtered. As an expert in the field may know, filtration can be carried out through different filters with different diameters, i.e. glass fiber membrane, filter papers with different diameters.

Finally, the product obtained after the filtration can be concentrated. Techniques of concentration of a composition are widely known to the person skilled in the art and any of them may be employed in the method of the invention, *i.e.* rotary evaporator.

The yeast extract recovered from the culture product can be further processed. Thus, the obtained yeast extract can be dried. The method of drying is not specifically limited, and any method which is usually conducted for preparing dry yeast extract can be adopted. The method of drying can be exemplified by the freeze dry method, the lyophilization method, heat dry method, or the like.

The oleaginous yeasts, the oleaginous yeasts biomass or the yeast extract obtained after the first method of the invention may be in the form of a composition. The composition may be solely composed of the oleaginous yeasts biomass or the yeast extract, or may contain another component such as a stabilizer and vehicles or carriers. The term "stabilizer" refers to a chemical that is used to prevent degradation. The term "vehicle" or "carrier" refers preferably to an inert substance. The functions of the carrier are to facilitate the incorporation of other components or compounds, to allow for better dosage and administration and/or to give consistency and shape to the composition.

### Biomass of the invention

As explained above, the oleaginous yeast biomass can be recovered from the culture product obtained after the first method of the invention. Thus, in a second aspect, the present invention relates to a biomass of an oleaginous yeast obtained by the first method of the invention (hereinafter "the biomass of the invention").

The culture medium used to obtain the biomass of the invention can or cannot comprise PO₄³⁻. When PO₄³⁻ is present in the culture medium, the ratio PO₄³⁻: Ca²⁺ is lower than 675. In a particular embodiment the ratio PO₄³⁻: Ca²⁺ is lower than 350, lower than 300, lower than 250, lower than 200, lower than 150, lower than 100, lower than 50, or lower than 25, preferably the ratio PO₄³⁻: Ca²⁺ is comprised between 30 and 350. In a particular embodiment, the ratio PO₄³⁻: Ca²⁺ is about 336.7, about 253.3, about 170 or about 33.33.

The terms "biomass" and "the ratio PO₄³⁻: Ca²⁺" have been defined or explained above, and these definitions are applicable to the biomass of the invention.

In a particular embodiment, the oleaginous yeast is selected from the group consisting of: *Yarrowia lipolytica*, *Cutaneotrichosporon oleaginosus* (previously known as *T. oleaginosus, Cryptococcus curvatus, Apiotrichum curvatum* or *Candida curvata*) *R. toruloides, Rhodotorula mucilaginosa, Rhodotorula mucilaginosa var. mucilaginosa, Rhodotorula terpenoidalis, Sporobolomyces alborubescens, Starmerella bombicola, Torulaspora delbruekii, Torulaspora pretoriensis*, *Lipomyces lipofer, L. starkey, Debaryomyces hansenii, Candida lipolytica, Candida glabrata, Candida tropicalis, Hansenula polymorpha, Hansenula californica, Hansenula beijerinckii, Geotrichum silvicola, Geotrichum vulgare* and *Williopsis saturnus.*

In a more particular embodiment, the oleaginous yeast is from the species *Y. lipolytica.* The terms "*Yarrowia*" and "*Yarrowia lipolytica*" have been defined or explained above, and these definitions are applicable to the biomass of the invention.

In a particular embodiment, the biomass obtained by the first method of the invention is enriched in lipid content. The term "biomass enriched in lipid content" as used herein, refers to a biomass of the oleaginous yeast having a lipid content higher than the lipid content of a biomass of reference. The biomass of reference would be a biomass of the same yeast cultured under standard conditions, for example using a medium with 10 to 20 g/L glucose as carbon source, with a C/N ratio between 10 and 20, under a temperature of incubation of 28 to 30°C, an agitation speed of 200 to 250 rpm and with a controlled pH of 5.0 to 5.5. In one embodiment, the biomass of the oleaginous yeast, particularly the biomass of *Y. lipolytica* enriched in lipid content has an improvement in the lipid content over the reference biomass of at least 10%, at least 20%, at least 30%, at least 40%, at least 50%, at least 60%, at least 70%, at least 80% at least 90%, at least 100%, at least 200%, at least 300% or more.

In a particular embodiment, a biomass enriched in lipid content refers to the biomass which has a lipid content higher than 30% w/w. The lipid content is expressed in "% w/w", which refers to the weight of lipid content in relation to total dry biomass. In a preferred embodiment, the biomass of the invention has a lipid content higher than 40% w/w. In a more preferred embodiment, the biomass of the invention has a lipid content higher than 50% w/w.

In a particular embodiment, the biomass of the invention enriched in lipid content is obtained by the first method of the invention where the culture medium comprises at least 20 g/L of total SCFAs.

The lipid content determination is not specifically limited, and any method which is usually conducted for determining the lipid content from a culture product can be adopted. In a particular embodiment, the total lipid content (% w/w) is fluorometrically determined. In another particular embodiment, the lipid content is gravimetrically determined.

In a particular embodiment, the biomass enriched in lipid content, preferably a biomass of *Y. lipolytica,* is obtained by a method wherein if the culture medium comprises PO₄³⁻, the ratio PO₄³⁻: Ca²⁺ is lower than 35 and wherein the culture medium comprises at least 20 g/L of total SCFAs.

### Method for enriching the lipid content of a biomass of an oleaginous yeast

In a third aspect, the invention relates to a method for enriching the lipid content of a biomass of an oleaginous yeast (hereinafter "the second method of the invention"), the method comprising culturing the oleaginous yeast with a culture medium comprising SCFAs as carbon source and Ca²⁺, wherein
- the culture medium does not comprise PO₄³⁻or
- the culture medium comprises PO₄³⁻and the ratio PO₄³⁻: Ca²⁺ is lower than 150 and wherein the culture medium does not comprise more than 2 g/L of glucose.

The terms "culture" or "cultivation" and "oleaginous yeast" have been described or explained in the first method of the invention, and these definitions are applicable to the second method of the invention.

In a particular embodiment, the oleaginous yeast is selected from the group consisting of: *Yarrowia lipolytica, Cutaneotrichosporon oleaginosus* (previously known as *T. oleaginosus, Cryptococcus curvatus, Apiotrichum curvatum* or *Candida curvata*) *R. toruloides, Rhodotorula mucilaginosa, Rhodotorula mucilaginosa var. mucilaginosa, Rhodotorula terpenoidalis, Sporobolomyces alborubescens, Starmerella bombicola, Torulaspora delbruekii, Torulaspora pretoriensis*, *Lipomyces lipofer, L. starkey, Debaryomyces hansenii, Candida lipolytica, Candida glabrata, Candida tropicalis, Hansenula polymorpha, Hansenula californica, Hansenula beijerinckii, Geotrichum silvicola, Geotrichum vulgare* and *Williopsis saturnus.*

In a more particular embodiment, the oleaginous yeast used in the second method of the invention is from the species *Y. lipolytica.*

The terms "*Yarrowia*" and "*Y. lipolytica*" have been defined or explained above, and these definitions are applicable to the second method of the invention.

The second method of the invention is able to achieve the lipid-increasing effect not only in cases of culturing wild-type strains of oleaginous yeasts (natural yeasts), but also in cases of culturing mutant oleaginous yeasts obtained from a mutagenic treatment. The terms "wild-type strain" and "mutant strain" have been defined or explained before, and these definitions are applicable to the second method of the invention.

The mutagenic treatment in the present invention is not specifically limited as long as it is a treatment that can cause a mutation in a part of gene(s) of a yeast. Any usual technique for use in the production of mutant strains of a yeast may be employed. For example, a mutagenic treatment onto a yeast can be carried out by treating the yeast with, as a mutagen, ultraviolet radiation, ionizing radiation, a nitrite, nitrosoguanidine or EMS.

The terms "biomass" and "carbon source" have been explained or defined above, and these definitions are applicable to the second method of the invention.

The second method of the invention comprises using SCFAs as the main carbon source. However, the culture medium may comprise other carbon source. The additional carbon source of the culture medium may be exemplified by one or more materials selected from the group consisting of glucose, sucrose, acetic acid, ethanol, molasses, spent sulphite liquor and the like, which, as a person skilled in the art may know, are used for routine cultivation of a microorganism. However, in a particular embodiment, the culture medium does not comprise other carbon source.

In a particular embodiment, if the culture medium comprises glucose as an additional carbon source, it does not comprise more than 2 g/L of glucose. In a more particular embodiment, the culture medium does not comprise more than 1 g/L, more than 0.75 g/L, more than 0.50 g/L, more than 0.25 g/L, more than 0.01 g/L of glucose. Additionally, the culture medium may comprise other components like a nitrogen source and an inorganic salt.

The nitrogen source may be either a nitrogen-containing inorganic salt or a nitrogen-containing organic material. For example, one or more materials selected from the group consisting of urea, ammonia, ammonium sulfate, ammonium chloride, ammonium phosphate, corn steep liquor (CSL), casein, yeast extract, peptone, and the like, can be used. Moreover, the inorganic salt can be exemplified by a phosphate component such as calcium superphosphate or ammonium phosphate, a potassium component such as potassium chloride or potassium hydroxide, a magnesium component such as magnesium sulfate or magnesium chloride, or the like. In addition, an inorganic salt of zinc, copper, manganese, ferrous ion, or the like, may also be used. Furthermore, the culture medium can comprise vitamins.

The terms "vitamin" or "essential nutrient" have been defined or explained above, and these definitions are applicable to the second method of the invention.

As mentioned above, the second method of the invention comprises culturing the oleaginous yeast with a culture medium comprising at least SCFAs as carbon source and Ca²⁺. Before inoculating the culture medium with oleaginous yeast, the second method of the invention can comprise a previous step which consist in the culture of the oleaginous yeast in a nutritive medium with the objective of generate an initial quantity of cells that will be used to inoculate the culture medium. Thus, in a particular embodiment, the second method of the invention comprises a previous step of culturing the yeasts until they reached the late exponential growth phase.

The terms "exponential growth phase" or "logarithmic growth phase", and "nutritive medium" have been defined or explained above, and these definitions are applicable to the second method of the invention.

In this previous step, the oleaginous yeasts are cultivated in a nutritive medium. The culture of the oleaginous yeast until it reached the exponential growth phase can be done in different nutritive mediums. For example, it can be used PDA, YEPD or YPD mediums. In a particular embodiment, the nutritive medium is YPD (yeast extract-peptone-dextrose), and the YPD nutritive medium can be liquid or solid.

In a particular embodiment, the culture of the oleaginous yeast with the culture medium is made at a temperature of between 20°C and 30°C (both ends included) in stirred conditions, and at an initial pH of between 5.0 - 8.0 (both ends included). In a more particular embodiment, the culture is done at 27°C and at initial pH of 6. The culture of the oleaginous yeast can be done in pH-uncontrolled culture conditions or in pH-controlled culture conditions. In a particular embodiment of the present invention, the culture of the oleaginous yeast is done in pH-uncontrolled culture conditions.

The term "stirred conditions" has been defined or described in the first method of the invention, and this definition is applicable to the second method of the invention.

The stirred conditions can be appropriately determined with consideration of the volume, the duration of the culture, and the initial concentration of the yeast. In a particular embodiment, stirred conditions are realised at approximately 10, 20, 30, 40, 50, 55, 60, 65, 70, 75, 80, 85, 90, 96, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 260, 270, 280, 290, 300, 310, 320, 350, 370, 400, 450, 500, 550, 600, 650, 700, 750, 750, 800, 850, 900, 1000 rpm, preferably the stirred conditions are realised at 170 rpm.

In a particular embodiment, stirred culture conditions can be realised by placing the culture in a shake flask, a rotary shaker, a stirred bioreactor, a flask placed on a shaker platform or a rotator. In a particular embodiment, stirred culture conditions are carried out with a rotary shaker.

In another particular embodiment, the oleaginous yeast is cultivated in the culture medium in stirred conditions until more than the 75-80% of the SCFAs were consumed, preferably until more than 85-90% of the SCFAs were consumed, more preferably until 95-100% of the SCFAs were consumed.

As an expert in the field may know, many different instrumental techniques can be used to determine the SCFAs concentration. In a particular embodiment, the SCFAs concentration in the culture medium is measured using the liquid chromatography (LC). In another particular embodiment, upon inoculation of the culture medium with the oleaginous yeast, an initial optical density (OD) at a wavelength of 600 nm is set of about 0.5, 0.6, 0.7, 0.8, 0.9, 1, 2, 3, 4, 5, 6, 7, preferably about 1 (equivalent to 0.45 g DW cells/L).

The carbon source in the culture medium according to the second method of the invention is one or more SCFAs. The term "SCFAs" has been defined or explained above, and this definition is applicable to the second method of the invention.

In a particular embodiment, the SCFAs of the culture medium are selected from the group consisting of acetic acid, propionic acid, butyric acid, valeric acid, hexanoic acid and combination thereof.

In a particular embodiment, the carbon source of the culture medium is a combination of SCFAs, preferably a combination of acetic acid, propionic acid, butyric acid, valeric acid and hexanoic acid. In a particular embodiment of the present invention, the culture medium comprises at least 10 g/L of total SCFAs. In a more particular embodiment, the culture medium comprises at least 20 g/L of total SCFAs.

In a particular embodiment, the concentration of the acetic acid is between 2 and 20 g/L, preferably between 2.5 and 10 g/L (all ends included).

In another particular embodiment, the concentration of the propionic acid is between 0.5 and 10 g/L, preferably between 1 and 4 g/L (all ends included).

In another particular embodiment, the concentration of the butyric acid is between 5 and 15 g/L, preferably between 5.5 and 9 g/L (all ends included).

In another particular embodiment, the concentration of the valeric acid is between 1.5 and 10 g/L, preferably between 2 and 5 g/L (all ends included).

In another particular embodiment, the concentration of the hexanoic acid is between 2 and 10 g/L, preferably between 2 and 5 g/L (all ends included).

The culture medium can or cannot comprise PO₄³⁻. When PO₄³⁻ is present in the culture medium, the ratio PO₄³⁻: Ca²⁺ is lower than 150. In a particular embodiment the ratio PO₄³⁻: Ca²⁺ is lower than 100, lower than 50, or lower than 25, preferably the ratio PO₄³⁻: Ca²⁺ is comprised between 0 and 35. In a particular embodiment, the ratio PO₄³⁻: Ca²⁺ is about 33.33.

The term "the ratio PO₄³⁻: Ca²⁺" has been defined or explained in the first method of the invention, and this definition is applicable to the second method of the invention. In a particular embodiment, the ratios PO₄³⁻: Ca ²⁺are obtained by adding Ca, preferably in the form of CaCl₂, and PO₄³⁻, preferably in the form of KH₂PO₄, to the culture medium. In a more particular embodiment, the culture medium is supplemented with a calcium concentration varying between 0.01 and 0.05 g/L, preferably 0.03 g/L (corresponding to 0.09 g/L of CaCl₂).

It will be understood that the concentrations of these components, while being present at concentration ratios as defined above, should also be present at concentrations that do not result in a substantially toxicity to the cells. The expression "concentration that does not result in a substantial toxicity to the cells" has been defined above, and this definition is applicable to the second method of the invention.

As a person skilled in the art understands, after the culture of the oleaginous yeast with the culture medium, or after the fermentation process, the oleaginous yeast or the biomass of the oleaginous yeast can be recovered from the culture product by a known method. The method of recovering the yeast or the biomass of the yeast from the culture product is not specifically limited, and any recovery method which is usually conducted for recovering a yeast from a culture product of the yeast can be adopted. The method of recovering the yeast or the biomass of the yeast from the culture product can be exemplified by a method of centrifuging and filtrating the culture product.

The terms "fermentation" and "culture product of the yeast" have been defined or explained above, and these definitions are applicable to the second method of the invention.

In addition to obtaining the oleaginous yeast biomass, a yeast extract can also be obtained from the culture product of the yeast. The term "yeast extract" has been defined or explained in the first method of the invention, and this definition is applicable to the second method of the invention. Techniques and conditions for preparing a yeast extract are widely known in the prior art, and any of them can be employed in the context of the present invention.

The preparation of the yeast extract can be exemplified by the autolysis method of lysing cell bodies with the aid of proteases that are naturally present in the yeast cells, the enzymatic degradation method of lysing cell bodies by adding an enzyme preparation derived from a microorganism or a plant, the hot water extraction method of lysing cell bodies by soaking cells in hot water for a fixed period of time, the acid/alkali decomposition method of lysing cell bodies by adding various types of acids or alkalis, the freezing and thawing method of disrupting cell bodies by conducting one or more times of freezing and thawing, the physical disruption method of disrupting cell bodies by a physical stimulation, or the like. The physical stimulation to be used in the physical disruption method can be exemplified by ultrasonic treatment, homogenization under high pressure, grinding by mixing with a solid material such as glass beads, or the like.

After the extraction process, the resulting product is filtered. As an expert in the field may know, filtration can be carried out through different filters with different diameters, i.e. glass fiber membrane, filter papers with different diameters. The term "extraction" has been defined or explained before, and this definition is applicable to the second method of the invention.

Finally, the product obtained after the filtration can be concentrated. Techniques of concentration of a composition are widely known to the person skilled in the art and any of them may be employed in the method of the invention, i.e. rotary evaporator.

As a person skilled in the art may know, the oleaginous yeast biomass having a high lipid content or the yeast extract can be further processed. Thus, the obtained oleaginous yeast biomass can be washed with distilled water one or more times, preferably more than 2 times, and can be dried. The method of drying is not specifically limited, and any method which is usually conducted for preparing dry yeast cells or dry biomass of yeast can be adopted. The method of drying can be exemplified by the freeze dry method, the lyophilization method, heat dry method, or the like.

The biomass of oleaginous yeast which has a high lipid content, or the yeast extract obtained by the second method of the invention may be in the form of a composition. The composition may be solely composed of the biomass of the oleaginous yeast, the dry yeast cells, or the yeast extract, or may contain another component such as a stabilizer and vehicles or carriers. The terms "stabilizer" and "vehicle" or "carrier" have been defined or explained in the first method of the invention, and these definitions are applicable to the second method of the invention.

### EXAMPLES

Having now generally described the invention, the same will be more readily understood through reference to the following examples, which are provided by way of illustration and are not intended to be limiting of the present invention.

The authors of the present invention have investigated the lipid accumulation in *Y*. *lipolytica* using SCFAs derived from the AF of wastes. In particular, yeast growth, SCFAs uptake and lipid content (% w/w) were studied on real and synthetic SCFAs-rich media. To elucidate the effect of Ca²⁺ and PO₄³⁻ in yeast metabolism, different PO₄³⁻: Ca²⁺ ratios were used.

### 1. Materials and Methods

### 1.1 Yeast strain and fermentation conditions

*Y. lipolytica* ACA DC 50109 (Agricultural University of Athens' culture collection) was selected due to its capacity to accumulate more than 20% w/w DW (Dourou et al., 2017, Applied Microbiology and Biotechnology 101, 7213-7226). Yeast was maintained at -80 °C in 30% v/v glycerol. The strain was kept prior to the fermentation experiments at 4 ± 1 °C in yeast extract-peptone-dextrose (YPD) agar medium (10 g/L yeast extract, 20 g/L peptone, 20 g/L glucose and 20 g/L agar) and was periodically sub-cultured. Colonies were pregrown by inoculating a colony in YPD liquid medium (same composition as mentioned above, except for the agar) and incubated overnight at 150 rpm and 27 °C in a rotary shaker until it reached the late exponential growth phase.

### 1.2 Fermentation media and conditions

Real digestates (RD) and synthetic media (SM) used in this investigation are compiled in Table 1. The anaerobic effluents obtained by Greses and co-workers (2020, Bioresource Technology 297, 122486) were centrifuged for 30 min at 5000 rpm (Heraeus, Megafuge 16, Thermo Scientific, FiberliteTM F15-6 × 100y fixed-angled rotor) in order to obtain the RD rich in SCFAs (acetic, propionic, butyric, valeric and caproic acid). After centrifugation, sterilization of the medium and particle removal was achieved by filtration.

In order to investigate the effect of Ca²⁺ and PO₄³⁻ in yeast growth and lipid content (% w/w), different PO₄³⁻: Ca²⁺ ratios in the range of 0 to 350 were used (specifically PO₄³⁻ : Ca²⁺ ratios of 336.7, 253.3, 170 and 33.33 were tested). These ratios were obtained by supplementing media with 0.03 g/L Ca²⁺ (corresponding to 0.09 g/L CaCl₂) and different amounts of KH₂PO₄ to achieved four different concentrations of total PO₄³⁻: 1 g/L, 5.1 g/L, 7.6 g/L and 10.1 g/L, respectively. The CaCl₂ concentration was calculated based on the results obtained by Nabais and co-workers (1988, Applied and Environmental Microbiology 54, 2439), while the PO₄³⁻ concentration was calculated based on the previous research (unpublished data).

SM 1 and 2 rich in SCFAs were prepared to replicate the same SCFAs concentration and profiles exhibited by RD 1 and 2. In all synthetic media, SCFAs were added to Delft medium (7.5 g/L (NH₄)₂SO₄, 14.4 g/L KH₂PO₄, 0.5 g/L MgSO4-7H₂O, 15 g/L SCFAs and 2 mL of trace metals solution) with different amounts of KH₂PO₄ to achieved four different concentrations of total PO₄³⁻: 0 g/L, 5.1 g/L, 7.6 g/L and 10.1 g/L. Likewise, 0.03 g/L Ca²⁺ (corresponding to 0.09 g/L CaCl₂) was added in all media to mimic the media conditions of RD, thus the PO₄³⁻:Ca ratios achieved in this media was 0, 170, 253.3 and 336.7, respectively.

Three replicates of each experiment were carried out in Baffled Erlenmeyer flasks of 250 mL. When inoculating fermentations, an initial optical density (OD) of 1 was set at 600 nm (OD600) (equivalent to 0.45 g DW cells/L). Each flask contained 100 mL of fermentation broth with an initial pH of 6.0 (no control pH was carried out). Flasks were incubated at 170 rpm and 27 °C in a rotary shaker until 95-100% of the SCFAs were consumed.

Samples were taken regularly to analyze yeast growth and SCFAs consumption. Lipid analysis was carried out at the end of the fermentation time.

### 1.3 Analytical methods

### 1.3.1 SCFAs measurement

Liquid chromatography was used to measure SCFAs concentration with an Agilent 1260 HPLC-RID (Agilent, Santa Clara, CA, USA) fitted with a Cation H Refill Cartridge Microguard column (Biorad, Hercules, CA, USA) and an Aminex HPX-87H ion exclusion column (300 × 7.8 mm I.D.) (Biorad). An elution in isocratic mode at a flow rate of 0.35 mL/min with a mobile phase of 5 mM H₂SO₄ was used. The injected sample volume was 20 µL. The detector and the oven were set at 35 °C and 50 °C, respectively.

### 1.3.2 Media compounds and yeast growth determination

The method of Vanadate-Molybdate was used to quantify the amount of PO₄³⁻ present in both SM and RD media. Total Ca was measured by ion chromatography (ICS 3000, Dionex) coupled with pre-columns and separation columns CG 16 and CS16 (3 mm ø) for cations. 35 °C was set as column temperature.

Cell growth of the cultures was measured at OD600 with Spectroquant^{®} Pharo 100 spectrophotometer. Dry biomass was calculated using a previously established standard curve, which correlates the OD of the culture with the yeast biomass production (g/L).

### 1.3.3 Lipid determination

Total lipid content (% w/w) was fluorometrically determined as described in Morales-Palomo and co-workers (2022, Biotechnology for Biofuels and Bioproducts 15, 37). Fluorescence intensity was determined at λex/em = 488/570 nm, ex/em slit = 10 nm (PerkinElmer^{®} LS 55 Fluorescence Spectrometer). Total areas were calculated with Origin (Pro), version 8.5 (OriginLab Corporation, Northampton, MA, USA) and used to obtain the quantum yield values of each sample. Additionally, a gravimetric extraction of each sample was carried out as described by (Llamas et al., 2020, Biomass and Bioenergy 138, 105553) in order to establish a standard curve between the lipid content, expressed as grams of lipid per grams of dry biomass (% w/w), and the quantum yield.

### 1.3.4 Data analysis

A parametric one-way ANOVA was used for the assessment of cell growth (OD600) and yeast lipid content (% w/w) (confidence interval 90%). Differences were considered significant at p-value < 0.05.

**Table 1. Composition of the different fermentation media used in this study.**

| Medium | SCFAs concentration* (g/L) | | | | | Total SCFAs |
|---|---|---|---|---|---|---|
| | Acetic | Propionic | Butyric | Valeric | Caproic | |
| SM 1 | 2.6 | 1.2 | 6.3 | 2.4 | 2.6 | 15.0 |
| SM 2 | 3.5 | 2.5 | 6.9 | 3.5 | 3.4 | 20.0 |
| RD 1 | 2.6 | 1.2 | 6.3 | 2.4 | 2.6 | 15.0 |
| RD 2 | 3.5 | 2.5 | 6.9 | 3.5 | 3.4 | 20.0 |

| | | | | | | |
|---|---|---|---|---|---|---|
| * The isobutyric and isovaleric values were negligible. | | | | | | |

### 2. Results

### 2.1 Effect of PO₄³⁻: Ca²⁺ ratio in yeast growth and lipid content (% w/w) in synthetic media

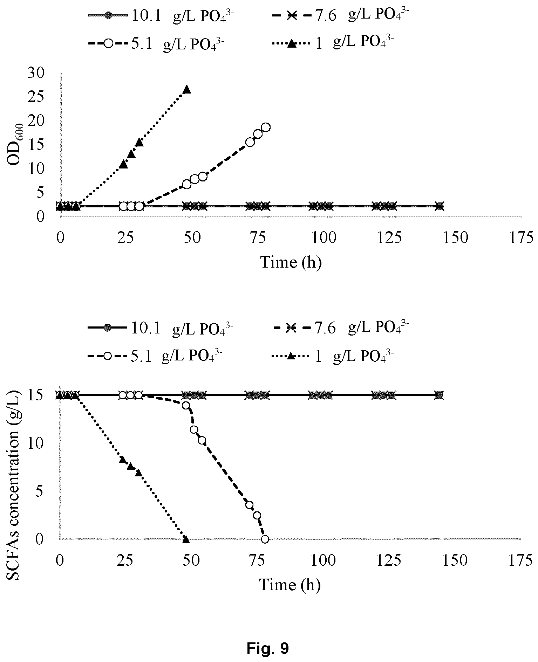

As it was previously studied, certain amounts of PO₄³⁻ could inhibit yeast growth. As seen in Fig. 9, the yeast barely showed a lag phase and was able to consume all the substrate in the medium with 1 g/L PO₄³⁻. Nevertheless, when the amount of PO₄³⁻ in the medium was 5.1 g/L, the yeast lengthened its lag phase up to 48 h and decreased its biomass production, and was unable to grow in the media when 7.6 g/L and 10.1 g/L PO₄³⁻ were used.

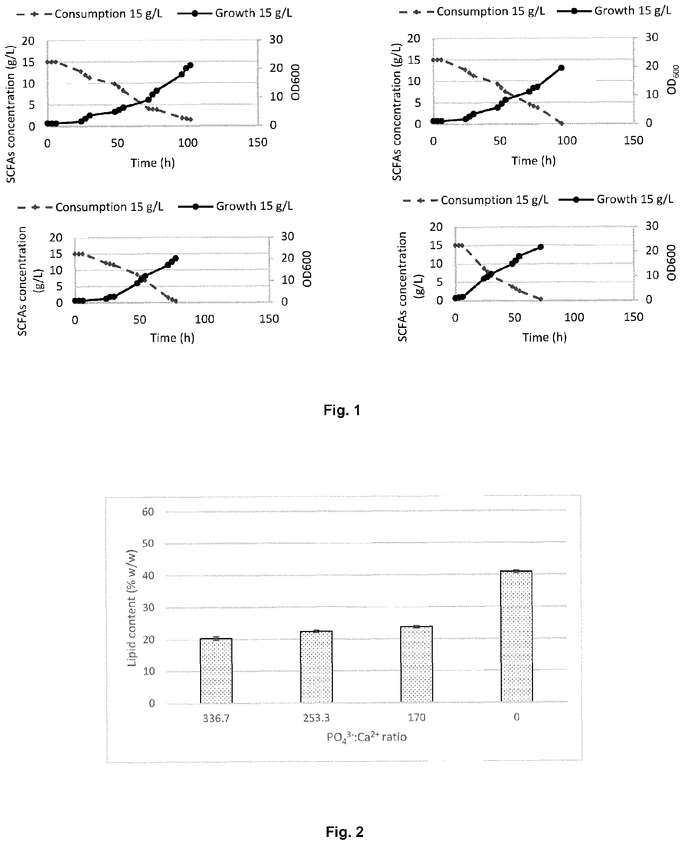

As was reported in the literature, PO₄³⁻ has a chelating activity on Ca, which is an important factor for yeast growth (Rachman et al., 2021, Suranaree Journal of Science & Technology 28, 1-7). Some studies show that Ca supplementation, in optimal concentrations, helps yeasts to overcome certain inhibitory barriers in presence of toxic substrates such as ethanol. In order to check the mentioned chelating activity, 0.09 g/L of CaCl₂ was supplemented (Nabais et al., 1988, Applied and Environmental Microbiology 54, 2439) to check if the yeast was able to grow in the same PO₄³⁻concentrations as those used in a previous study (unpublished data) (Section 2.2). As can be observed in Fig. 1, after the Ca²⁺ supplementation, no lag phase was observed in any case, contrary to what was previously seen in the unpublished article. Moreover, the fermentation time required to consume all the substrate was shorter as the PO₄³⁻: Ca²⁺ ratio decreased (decreased amount of PO₄³⁻). No significant differences in lipid content were observed when compared with the previous study (Fig. 2) (unpublished data). The results herein support the chelating effect of PO₄³⁻ over Ca²⁺, pointing out that large amounts of PO₄³⁻ (higher PO₄³⁻:Ca²⁺ ratios) could inhibit yeast growth.

### 2.2 Effect of PO₄³⁻:Ca²⁺ ratio in yeast growth and lipid content (% w/w) in real media

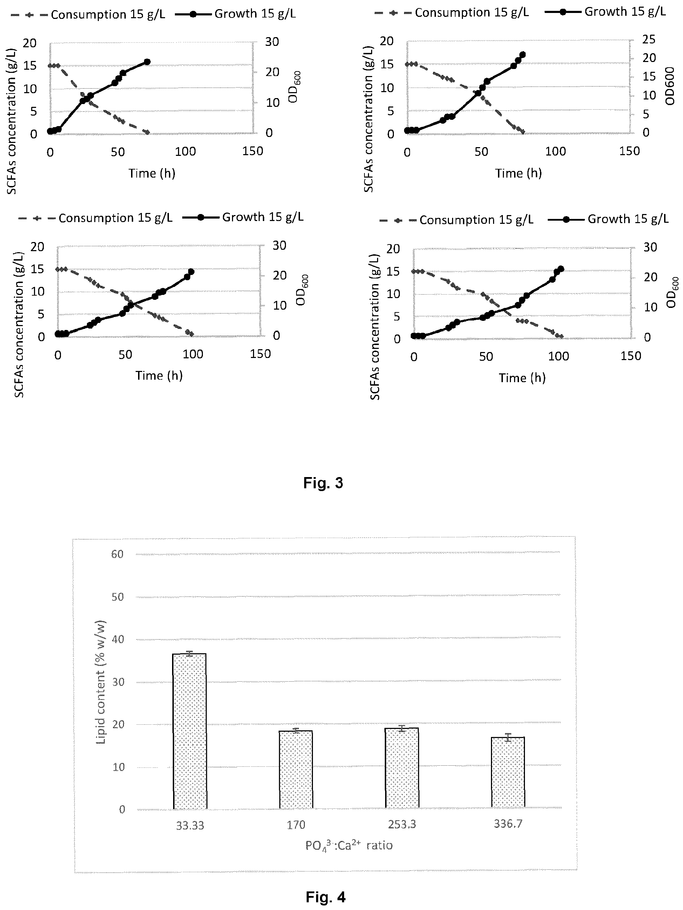

In order to verify the feasibility of carrying out the PO₄³⁻ limitation strategy in RD in presence of Ca²⁺, RD 1 with different PO₄³⁻: Ca ratios was used (section 2.2). As can be seen in Fig. 3, the yeast was able to grow in media with PO₄³⁻ : Ca²⁺ ratios of 253.3 and 336.7, contrary to what was seen in the previous study where there was no Ca²⁺ on the media (unpublished data). As seen in Fig, 4, with PO₄³⁻ : Ca²⁺ ratios of 253.3 and 336.7 similar results to those achieved in the previous investigation were observed when RD without Ca and 1 g/L and 5.6 g/L of PO₄³⁻ were used. Regarding the lipid content (% w/w) obtained with PO₄³⁻ : Ca²⁺ ratios of 253.3 and 336.7, the higher PO₄³⁻ : Ca²⁺ ratio (highest PO₄³⁻ concentration) led to a lower lipid content. According to these results, it can be concluded that greater availability of Ca²⁺ (lower PO₄³⁻ : Ca²⁺ ratio), would counteract the negative effect of PO₄³⁻ chelation and favour adaptation to the medium and yeast growth, although this does not influence lipid content (% w/w).

### 2.3 Effect of PO₄³⁻ : Ca²⁺ ratio in yeast growth and lipid content (% w/w) in synthetic media with 20 g/L FAs

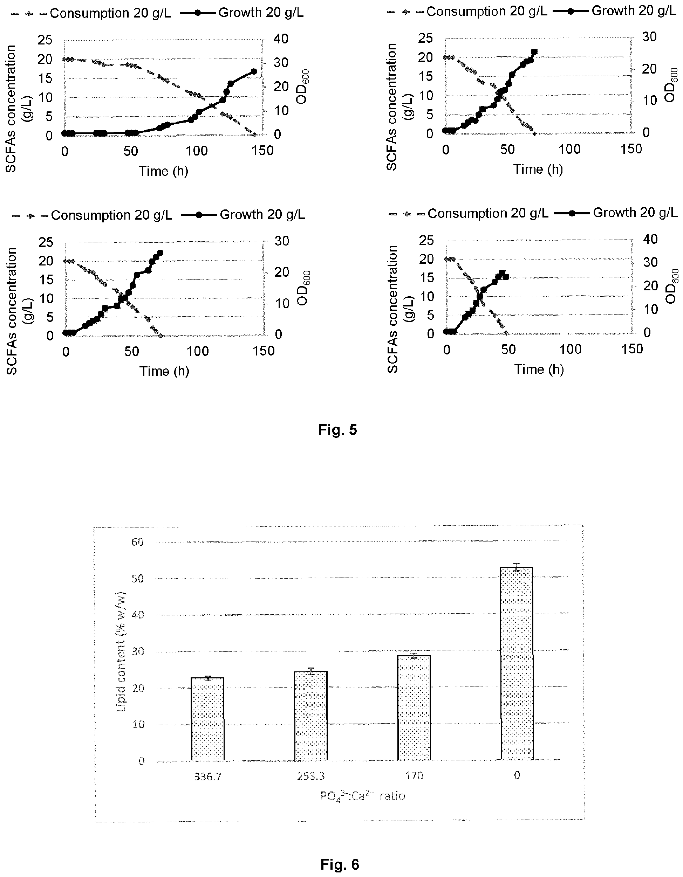

With the aim of increasing lipid content (% w/w) and testing the effect of PO₄³⁻ : Ca²⁺ ratio in the presence of an increased amount of SCFAs, SM that presented 20 g/L of total SCFAs were used. As expected, the yeast showed higher growth rates as the PO₄³⁻ : Ca²⁺ ratio decreased (PO₄³⁻ amounts decreased) (Fig. 5). Interestingly, the lipid content (% w/w) in the medium with PO₄³⁻ : Ca²⁺ ratio of 0 increased up to 52.66 % w/w (Fig. 6), providing a lipid yield of 0.31 (w/w). It should be noted that this lipid yield was similar to those reported in literature with glucose and xylose (0.32 g/g and 0.34 g/g, respectively), and in previous studies were nitrogen and phosphate-limitation strategies were used to increase lipid content (% w/w) (0.31 g/g in both cases) (unpublished data and Wang et al., 2020, Bioresource Technology 313, 123707). These results corroborate the capacity of Ca²⁺ to help yeast overcome certain inhibition barriers, even at concentration of SCFAs as high as 20 g/L. Furthermore, by allowing the initial concentration of SCFAs to be increased in the medium, it was possible to obtain a higher percentage of lipids, maintaining the maximum yields achieved with limitation by nitrogen and PO₄³⁻.

### 2.4 Effect of PO₄³⁻ : Ca²⁺ ratio in yeast growth and lipid content (% w/w) in real media with 20 g/L FAs

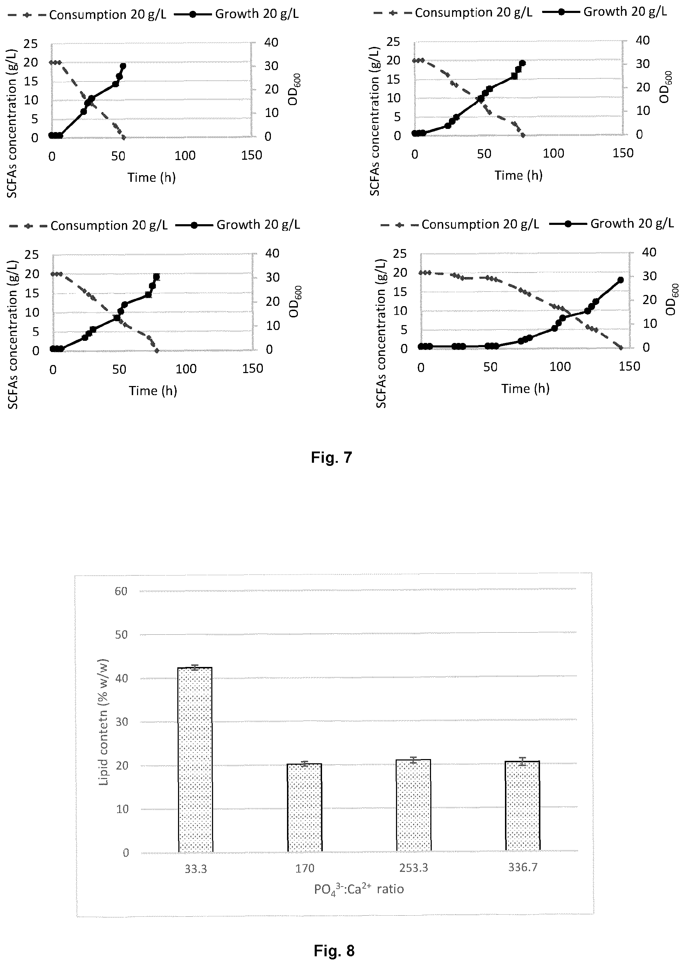

RD 2 with 20 g/L of SCFAs and different PO₄³⁻ : Ca²⁺ ratios was also tested to verify the feasibility of improving yeast lipid content (% w/w) in real media (section 2.2). As can be seen in Fig. 7, similar growth results to those obtained in SM with 20 g/L SCFAs were obtained. Regarding lipid content (% w/w), an increase from 36.55 % to 42.41 % w/w lipids DW was also observed in RD with PO₄³⁻ : Ca²⁺ ratio of 33.3 after increasing the amount of total SCFAs to 20 g/L, achieving a lipid yield of 0.29 (w/w). These results support the initial hypothesis that the lower the PO₄³⁻ : Ca²⁺ ratio (the more Ca²⁺ available in the medium not chelated by PO₄³⁻) the easier it is for the yeast to adapt to the media. Although it has be seen that lipid content (% w/w) is not directly affected by Ca²⁺, it indirectly enables the use of higher concentrations of SCFAs, which does result in increased lipid accumulation.

## Claims

1. A method for producing a biomass of an oleaginous yeast comprising culturing the oleaginous yeast with a culture medium comprising short chain fatty acids (SCFAs) as carbon source and calcium ion (Ca²⁺), wherein
- the culture medium does not comprise phosphate (PO₄³⁻) or
- the culture medium comprises PO₄³⁻ and the ratio phosphate/calcium ion (PO₄³⁻ : Ca²⁺) is lower than 675
and wherein the culture medium does not comprise more than 2 g/L of glucose.

2. The method according to claim 1, wherein the SCFAs are selected from the group consisting of acetic acid, propionic acid, butyric acid, valeric acid, hexanoic acid and combination thereof.

3. The method according to any one of claims 1 and 2, wherein the culture medium comprises at least 10 g/L of total SCFAs.

4. The method according to claim 3, wherein the culture medium comprises at least 20 g/L of total SCFAs.

5. The method according to any one of claims 1 to 3, wherein the ratio phosphate/calcium is comprised between 30 and 350.

6. The method of claim 1, wherein the oleaginous yeast is *Yarrowia lipolytica.*

7. A biomass of an oleaginous yeast obtained by the method according to any one of claims 1 to 6.

8. The biomass according to claim 7, wherein
- the culture medium does not comprise PO₄³⁻ or
- the culture medium comprises PO₄³⁻ and the ratio PO₄³⁻ : Ca²⁺ is lower than 150
and wherein the biomass is enriched in lipid content.

9. The biomass according to claim 8, wherein
- the culture medium does not comprise PO₄³⁻ or
- the culture medium comprises phosphate and the ratio PO₄³⁻ : Ca²⁺ is lower than 35
wherein the culture medium comprises at least 20 g/L of total SCFAs and wherein the oleaginous yeast is *Y. lipolytica*

10. The biomass according to any one of claims 8 or 9, wherein the biomass has a lipid content higher than 30% w/w.

11. A method for enriching the lipid content of a biomass of an oleaginous yeast, the method comprising culturing the oleaginous yeast with a culture medium comprising short chain fatty acids (SCFAs) as carbon source and calcium ion (Cₐ²⁺), wherein
- the culture medium not comprise phosphate (PO₄³⁻) or
- the culture medium comprises PO₄³⁻ and the ratio phosphate/calcium ion (PO₄³⁻ : Ca²⁺) is lower than 150
and wherein the culture medium does not comprise more than 2 g/L of glucose.

12. The method according to claim 11, wherein the SCFAs are selected from the group consisting of acetic acid, propionic acid, butyric acid, valeric acid, hexanoic acid and combination thereof.

13. The method according to claim 12, wherein the culture medium comprises at least 20 g/L of total SCFAs.

14. The method according to any one of claims 10 to 13, wherein the ratio phosphate/calcium is comprised between 0 and 35.

15. The method according to any one of claims 10 to 14, wherein the oleaginous yeast is *Y. lipolytica.*
